Europäisches Patentamt

European Patent Office

Office européen des brevets

(19)

(11) Publication number: **0 013 604**
**B1**

## EUROPEAN PATENT SPECIFICATION

(12)

(45) Date of publication of patent specification: **20.07.83**

(51) Int. Cl.³: **A 61 N 1/04**

(21) Application number: **80300035.5**

(22) Date of filing: **04.01.80**

(54) Flexible tip stiffening stylet for body implantable lead.

(30) Priority: **05.01.79 US 1203**

(43) Date of publication of application:
**23.07.80 Bulletin 80/15**

(45) Publication of the grant of the patent:
**20.07.83 Bulletin 83/29**

(84) Designated Contracting States:
**CH DE FR GB IT NL SE**

(56) References cited:
**DE - A - 2 539 553**
**FR - A - 2 119 261**
**US - A - 4 136 701**

(73) Proprietor: **MEDTRONIC, INC.**
**3055 Old Highway Eight P.O. Box 1453**
**Minneapolis Minnesota 55440 (US)**

(72) Inventor: **Dutcher, Robert G.**
**1310 Pierce Terrace, N.E.**
**Columbia Heights Minnesota 5542 (US)**
Inventor: **Upton, James E.**
**1629 23rd Avenue N.W.**
**New Brighton Minnesota 55112 (US)**

(74) Representative: **Parker, Jeffrey et al,**
**Frank B. Dehn & Co. Imperial House 15-19**
**Kingsway**
**London, WC2B 6UZ (GB)**

Courier Press, Leamington Spa, England

Flexible tip stiffening stylet for body implantable lead

This invention relates to a stylet for use with a lead bearing an electrode for electrically connecting an organ inside a living animal body to an electrical device. Notwithstanding its various uses, this invention will be described for use in an endocardial pacing and sensing lead for connecting an artificial cardiac pacemaker to cardiac tissue.

Endocardial pacing and sensing leads of a known type comprise one or more lengths of hollow, coiled wire conductor encased within a suitable insulating material, such as silicone rubber, that is substantially inert to body fluids and tissues, a hollow connector pin attached to the proximal end of each of the conductors, and an electrically conductive electrode at the distal end of each of the conductors adapted to be placed in contact with the endocardium of the patient. A lumen extends through each pin and the corresponding lengths of coiled wire conductor to the electrode at the distal ends thereof and receives a stiffening stylet of cylindrical corrosion resistant wire for imparting stiffness to the lead to facilitate its advancement through the venous system of the patient and into the apex of the right ventricle. With the stylet removed from the lead, the lead is very flexible and difficult to so advance. Further details of the construction and utility· of such endocardial pacing leads may be obtained by reference to US—A—3,348,584 and US—A—4,046,151.

An improved cardiac pacing lead, described, for example, in DE—A—2 539 553, employing a rigid helix with a sharp tipped distal end adapted to be screwed into the endocardium can be lodged in and permanently secured to or removed from body tissue without the use of bulky sleeves or catheter introducers to protect the patient's veins and tricuspid valve from snagging on the sharp tip of the helix. In such an improved lead the tissue securing means is a helix with a piston member fixed to its proximal end and positioned in a chamber within the electrode body. A stylet having a knob at its proximal end is passed through a lumen in the lead which communicates with the opening in the proximal end of the lead body such that the distal end of the stylet which is shaped in the form of a screwdriver head, is engageable with a slot in the head of the piston means. The stylet may be rotated after the distal end of the lead is positioned near the endocardial tissue and when the stylet is rotated the piston means is caused to screw the helix out of the distal opening in the electrode lead and into the endocardial and myocardial tissue to secure the electrode.

It is an object of the present invention to provide an improved stylet for an implantable lead to increase the effectiveness in the placement of the endocardial leads of the kind mentioned above. This object is achieved by the invention as claimed. A feature of the present invention is the provision of a stylet adapted to be extremely flexible in the vicinity of the distal end of the lead to increase the flexibility of the lead without detracting from the ability of the stylet to transmit torque from its proximal end to its distal end. The improved structure facilitates the transmission of torque by the stylet when the stylet and lead are sharply bent. The improved structure also permits the stylet and lead to be used where it is necessary to pass them through a sharp bend during insertion. Additionally, the improved flexible stylet may be inserted with less chance of snagging the helical coil conductor of the lead and perforating either the lead or surrounding tissue. The improved structure described also provides an improved screwdriver tip for inserting the electrode into the tissue into which it is to be lodged.

A specific embodiment of the invention is now described by way of example only with reference to the accompanying drawings, in which:—

Figure 1 is a view of a preferred embodiment of body implantable lead and insertion stylet showing in phantom outline form a thinned portion of the stylet;

Figure 2 is an enlarged side view of the distal end of the stylet of Figure 1 showing the variations in diameter;

Figure 3 is an enlarged top view of the distal end portion of the stylet; and

Figure 4 is an enlarged view of the proximal end portion of the stylet.

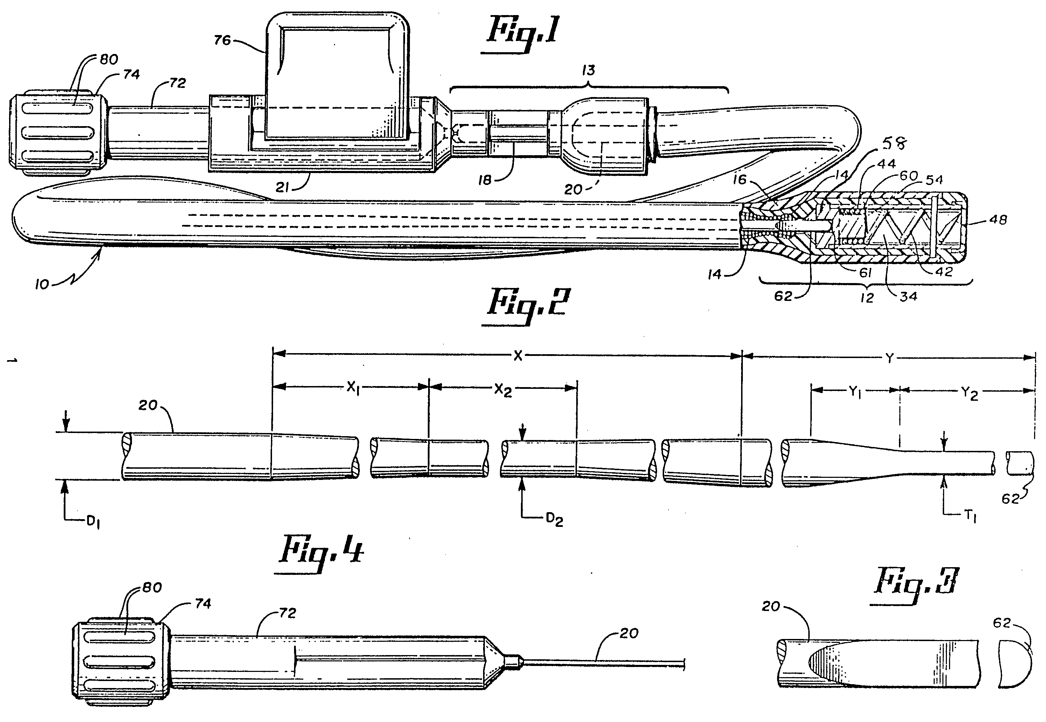

Referring now to the preferred embodiment of the invention depicted in Figure 1, there is shown an intravascular endocardial lead comprising an elongated lead 10, a distal electrode end portion 12, and a proximal terminal end portion 13. The lead, in unipolar configuration, comprises a closely wound, coiled conductor 14 in the form of a spring spirally wound about and along the axis of the conductor. The spring coil 14 extends through the length of lead 10 in a lumen of a jacket or sleeve 16 of electrically insulating material.

A tissue securing member in the form of a relatively rigid circular corkscrew or helix 42 is provided having a proximal end 44 of several closely wound turns located in the chamber 34 toward the proximal end thereof. Helix 42 has a sharpened tip 48.

The stylet used in the preferred embodiment of the invention disclosed herein is shown in detail in Figures 2 and 3. The stylet is made of a corrosion resistant steel, such as type 304 stainless steel wire, having a typical nominal diameter over most of its length of approximately 0.4 mm. This dimension is represented on Figure 2 as D1, and corresponds to the diameter of prior art stylets conventionally used in positioning leads of the type generally shown in Figure 1. As shown in Figure 2, the stylet disclosed herein has a reduced diameter portion near the distal end to facilitate transmission of rotational torque by the stylet in situations where the stylet and lead assembly are sharply bent in the vicinity of the distal end of the lead.

Attempts to achieve adequate transmission of rotational torque through a sharply bent stylet have included providing cylindrical stylets having uniform reduced diameters over their entire length. Those attempts have been unsuccessful since the reduction of the diameter of the entire stylet tends to weaken it unnecessarily and to make transmission of significant rotational torque difficult. In the stylet shown in Figure 2, the reduced diameter portion is provided only in the area which is subject to the extreme bending. This extreme bending can be expected, for example, in cases where the lead 10 is implanted in the right atrium of the heart, rather than the apex of the right ventricle. Another situation where the stylet is of utility is in a ventricular implantation where the tricuspid valve between the right atrium and right ventricle places a relatively sharp bend in the lead. In both situations, the reduced diameter portions of the stylet are located only in the vicinity of the bend. In such situations, the improved stylet can be inserted into the lead with a lessened risk of snagging at the bend and consequent damage to the lead and surrounding tissue. After insertion of the stylet, its improved structure provides for adequate transmission of rotational torque to permit implantation.

In the preferred embodiment of the improved stylet for use with a cardiac pacing lead 10, the length of the stylet from its screwdriver tip portion 62 to the proximal end of the stylet is slightly over 660 mm. As shown in Figure 2, the reduced diameter portion is provided near the distal end. Typical dimensions for distances X and Y are 100 mm and 12 mm respectively. The smooth transition between the diameter D1 and the diameter D2 is indicated by the dimension X1, which may typically be in the range of 19 mm, while the actual reduced diameter portion, indicated by D2, may typically be approximately 64 mm long, with a diameter of 0.33 mm. The distal end of the stylet is formed into a screwdriver tip 62, having a length Y2 of typically 3.8 mm and a tapered length Y1 of 0.75 mm typically. As shown in Figure 3, the tip 62 has a radius of 0.2 mm and a thickness T1 as viewed in Figure 2 of 0.2 mm.

In some applications, it may be necessary to utilize a standard uniform diameter stylet to stiffen the lead to facilitate insertion of the lead through the venous system and into the chamber of the heart where the helix 42 is desired to be implanted. After the lead is inserted and positioned, the insertion stylet is removed and the flexible tip stylet may then be inserted and utilized for the remainder of the implanting process. The flexible tip portion of the stylet makes it particularly useful for working the stylet around tight corners without damage to the lead. In some applications, it is particularly desirable to use the flexible tip stylet during insertion, since the flexibility of the stylet makes the lead less likely to perforate the ventricle, or to inhibit working the lead around corners.

There is also provided in chamber 34 at the distal end of lead 10, a member 54 which takes the form of a piston having a generally circular cross section and has a proximal end at which is located a slotted head 58 and a distal end portion 60 which is somewhat smaller in cross sectional diameter than head 58. Head 58 has a slot 61 in the proximal end thereof and is adapted to receive the distal end of stylet 20 which terminates at its distal end in a screwdriver tip 62. When flexible tipped stylet 20 is fully inserted into lead 10 through pin 18, its screwdriver tip portion 62 is firmly seated in slot 61.

An insertion member 21, is shown in Figure 1 mounted on pin 18 connected to the proximal end portion of lead 10. The insertion tool 21 is disclosed in a copending application No. 80300036.3 (EP—A—13605) entitled Stylet Insertion Assembly for Body Implantable Lead, filed on the same date as the present application.

Rotation of knob 74 and shaft 72 directly transmits rotational torque to stylet 20. The screwdriver blade tip 62 of stylet 20 engages slot 61 in piston 54 and in turn rotates the pointed tip 48 of the helix 42 of the lead to advance it into the body tissue against which the distal tip of the lead was positioned when the rotation of helix 42 was commenced.

Lead 10 may be positioned in the appropriate position in the heart either by using a conventional stylet which is then removed or using the flexible stylet 20 disclosed herein. After the lead is positioned, the screwdriver tip 62 of the stylet engages slot 61 of piston 54. The shaft 72 of the insertion tool 21 is then inserted into the guide chamber of guide 76 and guide 76 is fitted over the pin 18 and the proximal end of lead 10. Knob 74 is then rotated in the correct direction to cause helix 42 to advance it into the tissue against which the distal end of lead 10 was placed.

Although a unipolar lead design has been illustrated in the description of the preferred embodiment, it will be understood that bipolar leads (that is a lead carrying two electrodes and conductors) may as readily utilize the novel structure of the present invention. It should be understood that although the lead 10 has been described for use in a cardiac pacing system, lead 10 could as well be applied to other types of body stimulating systems.

**Claims**

1. A stylet insertable into a body-implantable tubular lead (10) and means (72, 74) attached to the stylet at the proximal end for effecting rotation of the stylet within the lead, said lead (10) having at its distal end (12) an electrode head including an electrode adapted to receive and/or supply electrical signals from or to body tissue and helical securing means (42) adapted to secure the electrode head to tissue within the body, the helical securing means being coupled to a rotatable member (59) disposed within a chamber (34) in the electrode head, the stylet being of a substantially uniform first diameter (D1) over most of its length and the distal end of the stylet being flattened into a screwdriver like tip (62) adapted for engagement with a slot (61) on the rotatable member whereby rotation of the stylet causes the helical means (42) to rotate and extend from the chamber and penetrate adjacent body tissue, characterised in that the stylet has a portion of reduced diameter towards the distal end thereof where the stylet gradually inwardly tapers from said first diameter (D1) to a second, reduced diameter (D2), and the stylet gradually tapers outwardly from said second diameter to a third and greater diameter at the distal end, the reduced diameter portion being short relative to the total length of the stylet, and being located in that region of the stylet which is prone to sharp bending when the helical securing means is being actuated, whereby transmission of rotational torque to the rotatable member by the stylet may be facilitated and is substantially unimpaired when the stylet is sharply bent in said region, the portion having said third diameter being short to enable ready negotiation of the stylet tip around a said sharp bend in use.

2. A stylet according to claim 1 wherein the length of the portion of reduced diameter (D2) is substantially 64 mm and the total length of the stylet is substantially 660 mm.

3. A stylet according to claim 1 or 2 wherein the stylet begins to taper inwardly at a point substantially 112 mm from the distal end.

4. A stylet according to any of the preceding claims wherein the inward tapering extends over substantially 19 mm.

5. A stylet according to any of the preceding claims in which the first diameter (D1) is substantially 0.4 mm and the second diameter (D2) is substantially 0.33 mm.

6. A stylet according to any preceding claim wherein the thickness of said screwdriver tip is substantially 0.2 mm.

7. A stylet according to any preceding claim wherein the length of said screwdriver tip (62) is substantially 3.8 mm.

8. A stylet according to any preceding claim wherein the screwdriver tip has a blade portion which is rounded across its width to a 0.2 mm radius.

9. A stylet according to any preceding claim wherein the transition between the screwdriver tip portion and the portion of the stylet having said third diameter occurs over substantially 0.75 mm.

**Revendications**

1. Un stylet insérable dans un conducteur tubulaire (10) implantable dans le corps et des moyens (72, 74) fixés au stylet à l'extrémité proximale de celui-ci pour effectuer une rotation du stylet à l'intérieur du conducteur, ledit conducteur (10) ayant à son extrémité distale (12) une tête formant une électrode adaptée pour recevoir et/ou fournir des signaux électriques provenant du tissu du corps ou dans le tissu du corps, et des moyens de fixation hélicoïdaux (42) adaptés pour fixer la tête de l'électrode au tissu à l'intérieur du corps, les moyens de fixation hélicoïdaux étant couplés à un élément rotatif (59) disposé dans une chambre (34) dans la tête de l'électrode, le stylet ayant un premier diamètre (D1) à peu près uniforme sur la plus grande partie de sa longueur et l'extrémité distale du stylet étant aplatie pour former une pointe (62) analogue à celle d'un tournevis et adaptée pour coopérer avec une fente (61) sur l'élément rotatif, grâce à quoi la rotation du stylet astreint les moyens hélicoïdaux (42) à tourner et à s'étendre depuis la chambre et à pénétrer dans le tissu adjacent du corps, caractérisé en ce que le stylet comporte une partie de diamètre réduit vers son extrémité distale où le stylet converge progressivement vers l'intérieur depuis le premier diamètre (D1) jusqu'à un second diamètre réduit (D2), et le stylet présentant une forme convergeant graduellement vers l'extérieur depuis ledit second diamètre vers un troisième et plus grand diamètre à l'extrémité distale, la partie de diamètre réduit étant relativement courte par rapport à la longueur totale du stylet, et étant située dans la région de celui-ci qui est sujette à former des coudes prononcés lorsque les moyens hélicoïdaux de fixation sont actionnés, de sorte que la transmission du couple de rotation à l'élément rotatif par le stylet peut être facilitée et est à peu près non gênée lorsque le stylet est plié de façon prononcée dans ladite région, la partie présentant ledit troisième diamètre étant courte pour permettre un passage facile de la pointe du stylet, en service, autour dans coude prononcé.

2. Un stylet suivant la revendication 1, dans lequel la longueur de la partie (D2) de diametre réduit est à peu près de 64 mm et la longueur totale du stylet est à peu près de 660 mm.

3. Un stylet suivant la revendication 1 ou 2 dans lequel le stylet commence à converger vers l'intérieur en un point situé à peu près à 112 mm de l'extrémité distale.

4. Un stylet suivant l'une quelconque des revendications précédentes, dans lequel la convergence vers l'intérieur s'etend sur environ 19 mm.

5. Un stylet suivant l'une quelconque des revendications précédentes, dans lequel le premier diametre (D1) est à peu près de 0,4 mm et le second diametre (D2) est à peu près de 0,33 mm.

6. Un stylet suivant l'une quelconque des revendications précédentes, dans lequel l'épaisseur de ladite pointe de tournevis est d'à peu près 0,2 mm.

7. Un stylet suivant l'une quelconque des revendications précédentes, dans lequel la longueur de ladite pointe (62) de tournevis est à peu près de 3,8 mm.

8. Un stylet suivant l'une quelconque des revendications précédentes, dans lequel la pointe du tournevis présente une lame qui est arrondie suivant sa largeur à un rayon de 0,2 mm.

9. Un stylet suivant l'une quelconque des revendications précédentes, dans lequel la transition entre la pointe de tournevis et la partie du stylet présentant ledit troisième diamètre est assurée sur à peu près 0,75 mm.

## Patentansprüche

1. Mandrin, der in eine im Körper implantierbare rohrförmige Leitung (10) einsetzbar ist und an dessen proximalem Ende Mittel (72, 74) zum Drehen des Mandrins innerhalb der Leitung angebracht sind, wobei die Leitung (10) an ihrem distalen Ende (12) mit einem Elektrodenkopf versehen ist, der eine zur Aufnahme von elektrischen Signalen von und/oder zur Zuführung von elektrischen Signalen zu Körpergewebe geeignete Elektrode und eine wendelförmige Befestigunsanordnung (42) aufweist, mittels deren sich der Elektrodenkopf am Gewebe innerhalb des Körpers festlegen läßt, wobei die wendelförmige Befestigungsanordnung mit einem drehbaren Organ (59) gekuppelt ist, das innerhalb einer Kammer (34) in dem Elektrodenkopf angeordnet ist, und wobei der Mandrin einen im wesentlichen gleichförmigen ersten Durchmesser (D1) über den größten Teil seiner Länge hat und das distale Ende des Mandrins zu einer schraubenzieherartigen Spitze (62) abgeflacht ist, die mit einem Schlitz (61) des drehbaren Organs in Eingriff bringbar ist, so daß eine Drehung des Mandrins die wendelförmige Anordnung (42) veranlaßt, sich zu drehen und aus der Kammer herausfahren sowie in benachbartes Körpergewebe einzudringen, dadurch gekennzeichnet, daß der Mandrin in Richtung auf sein distales Ende hin einen Abschnitt von vermindertem Durchmesser aufweist, im Bereich dessen sich der Mandrin von dem ersten Durchmesser (D1) allmählich nach innen auf einen zweiten verminderten Durchmesser (D2) verjüngt, und daß sich der Mandrin von dem zweiten Durchmesser allmählich nach außen auf einen dritten und größeren Durchmesser an dem distalen Ende erweitert, wobei der Abschnitt mit vermindertem Durchmesser kurz mit Bezug auf die Gesamtlänge des Mandrins und in demjenigen Bereich des Mandrins angeordnet ist, wo es zu einem scharfen Abbiegen kommen kann, wenn die wendelförmige Befestigungsanordnung betätigt wird, wodurch die Übertragung von Drehmoment von dem Mandrin auf das drehbare Organ erleichtert wird und im wesentlichen unbeeinträchtigt bleibt, wenn der Mandrin in diesem Bereich scharf abgebogen wird, wobei der den dritten Durchmesser aufweisende Abschnitt kurz ist, um bei Anwendung ein leichtes Herumführen der Mandrinspitze um die scharfe Abbiegung zu gestatten.

2. Mandrin nach Anspruch 1, dadurch gekennzeichnet, daß die Länge des Abschnittes mit vermindertem Durchmesser (D2) etwa 64 mm und die Gesamtlänge des Mandrins etwa 660 mm betragen.

3. Mandrin nach Anspruch 1 oder 2, dadurch gekennzeichnet, daß sich der Mandrin an einer etwa 112 mm von dem distalen Ende entfernten Stelle nach innen zu verjüngen beginnt.

4. Mandrin nach einem der vorhergehenden Ansprüche, dadurch gekennzeichnet, daß sich die nach innen gerichtete Verjüngung über etwa 19 mm erstreckt.

5. Mandrin nach einem der vorhergehenden Ansprüche, dadurch gekennzeichnet, daß der erste Durchmesser (D1) etwa 0,4 mm und der zweite Durchmesser (D2) etwa 0,33 mm betragen.

6. Mandrin nach einem der vorhergehenden Ansprüche, dadurch gekennzeichnet, daß die Schraubenzieherspitze etwa 0,2 mm dick ist.

7. Mandrin nach einem der vorhergehenden Ansprüche, dadurch gekennzeichnet, daß die Schraubenzieherspitze (62) etwa 3,8 mm lang ist.

8. Mandrin nach einem der vorhergehenden Ansprüche, dadurch gekennzeichnet, daß die Schraubenzieherspitze einen Schaufelabschnitt aufweist, der über seine Breite hinweg auf einen Radius von 0,2 mm abgerundet ist.

9. Mandrin nach einem der vorhergehenden Ansprüche, dadurch gekennzeichnet, daß der Übergang zwischen dem Schraubenzieherspitzenabschnitt und dem den dritten Durchmesser aufweisenden Abschnitt des Mandrins über etwa 0,75 mm erfolgt.

*Fig.1*

*Fig.2*

*Fig.3*

*Fig.4*